# EUROPÄISCHE PATENTSCHRIFT

(11) **EP 1 295 900 B1**
(45) Veröffentlichungstag und Bekanntmachung des Hinweises auf die Patenterteilung: **06.12.2006**
(21) Anmeldenummer: 02028222.4
(22) Anmeldetag: 23.06.1994
(51) Int. Cl.: C08G 59/14, C08G 59/42, C08G 59/04, C08F 290/14

(54) **Verwendung von Epoxyacrylaten**
Uses of epoxy-acrylates
Utilisations d'époxy acrylates

(30) Priorität: 02.07.1993 CH 200393
(43) Veröffentlichungstag der Anmeldung: 26.03.2003
(62) Teilanmeldung aus: 94810375.9
(73) Patentinhaber: Huntsman Advanced Materials (Switzerland) GmbH, 4057 Basel (CH)
(72) Erfinder: Roth, Martin Dr., 4434 Hölstein (CH); Salvin, Roger Dr., 79576 Weil am Rhein (DE); Meier, Kurt Dr., 4106 Therwil (CH); Sailer, Bernhard Dr., 4058 Basel (CH); Wiesendanger, Rolf Dr., 4125 Riehen (CH)
(74) Vertreter: Hoffmann, Daniele

(56) Entgegenhaltungen:
- EP-A- 0 167 051
- EP-A- 0 321 824
- WO-A-89/07785
- FR-A- 2 220 563
- FR-A- 2 320 961
- DATABASE WPI Section Ch, Week 198932 Derwent Publications Ltd., London, GB; Class A21, AN 1989-232343 XP002261807 & JP 01 168722 A (ASAHI-CIBA KK), 4. Juli 1989 (1989-07-04)

## Beschreibung

Die Erfindung betrifft die Verwendung höhermolekularer Epoxyacrylate in Photoresistformulierungen und die Anwendung dieser Formulierungen vor allem auf dem Gebiet der Leiterplatten, zum Beispiel als Lötstoppresist oder als Primärresist (Ätzresist oder Galvanoresist) und der Druckplatten.

Epoxyacrylate sind in grosser Zahl bekannt und werden unter anderem auch in Zusammensetzungen verwendet, die als Photoresistformulierungen dienen.

So beschreibt z.B. die EP 0 273 729 Zusammensetzungen für Lötstoppresists, die Umsetzungsprodukte aus Epoxy-novolakharzen mit Acrylsäure und cyclischen Carbonsäureanhydriden enthalten. Sie sind wässrig-alkalisch entwickelbar und weisen eine gute Wärmebeständigkeit und Photoempfindlichkeit auf. Allerdings lässt die Chemikalienbeständigkeit noch zu wünschen übrig.

Die EP 0 418 011 offenbart Zusammensetzungen für Lötstoppresists, ebenfalls basierend auf Reaktionsprodukten von Epoxy-kresolnovolaken mit Acrylsäure und cyclischen Dicarbonsäureanhydriden, wobei 0,4 bis 0,9 Äquivalente Acrylsäure pro Äquivalent Epoxygruppe eingesetzt werden, so dass das Endprodukt zugleich Säure- und Epoxygruppen im gleichen Molekül aufweist. Dadurch wird in der Applikation eine zweite thermische Vernetzungsreaktion zwischen diesen beiden Funktionalitäten ermöglicht. Problematisch ist hier jedoch neben der Herstellung der Produkte (Gefahr der Gelierung bei der Reaktion mit dem Anhydrid) die Lagerstabilität, da bereits bei Raumtemperatur eine bestimmte Reaktivität der derartige Umsetzungsprodukte enthaltenden Formulierung vorhanden ist. Generell sind all diese genannten Epoxyacrylate relativ niedermolekular.

Photochemisch oder thermisch gehärtete Epoxyacrylate, die sich von niedermolekularen Epoxyharzen und Epoxynovolaken ableiten, sind zwar für ihre guten thermischen und mechanischen Eigenschaften sowie ihre gute chemische Beständigkeit gegen agressive Chemikalien bekannt. Allerdings lassen aber die Klebrigkeit und die Kantendeckung der mit diesen Systemen erhaltenen Resistfilme auf Leiterzügen wegen der relativ niedrigen Molmasse zu wünschen übrig. Man ist deshalb in der Anwendung vielfach gezwungen, diese Nachteile durch den Zusatz von hochmolekularen Bindemittelpolymeren zu umgehen. Diese weisen normalerweise keine funktionellen Acrylatgruppen auf und reagieren bei der photochemischen oder thermischen Härtung nicht mit, das heisst, sie werden als "passive" Bestandteile im Netzwerk nicht eingebaut und führen somit zu einer Verdünnung der Netzwerkdichte. Dies hat wieder eine ungünstige Beeinflussung speziell der chemischen Beständigkeit und der elektrischen Eigenschaften von prozessierten Resistschichten zur Folge. Ausserdem sinkt die Photoempfindlichkeit infolge der "Verdünnung" der Acrylatgruppen. Durch die Verwendung von hochmolekularen Bindemittelpolymeren weisen diese Zusammensetzungen bereits bei relativ niedrigem Festkörpergehalt eine hohe Viskosität auf und führen daher oft zu grossen Problemen bei der Beschichtung.

Batzer und Zahir (J. Appl. Polym. Sci., 19, 609 (1975)) beschreiben die Postglycidylisierungs-reaktion eines niedermolekularen, flüssigen Bisphenol A-diglycidylethers. Die US 4 623 701 beschreibt postglycidylisierte Epoxyharze und ihre Härtung mit diversen Epoxyhärtem, und die US 4 074 008 offenbart photovernetzbare Epoxyharze mit mehr als 2 Epoxygruppen im Molekül, wobei mindestens 2 davon aus einer Postglycidylisierungs-reaktion stammen. Die in der Molekülkette angeordneten photovemetzbaren Gruppen stellen α,β-ungesättigte Carbonylsysteme dar (Chalcongruppen). (Meth)acrylgruppen enthaltende Derivate sind dort jedoch keine beschrieben. Es ist ferner bekannt, dass die Photoempfindlichkeit des nach einem 2+2-Cycloadditons-mechanismus lichtvernetzbaren α,β-ungesättigten Carbonylsystems im Vergleich zur Photopolymerisation von Acrylaten wesentlich geringer ist.

In der japanischen Patentanmeldung Kokai Hei 04-294352 werden Epoxynovolakharze durch Umsetzung mit einer ungesättigten Monocarbonsäure und anschliessend mit einem ungesättigten Anhydrid einer Polycarbonsäure modifiziert und in photoempfindlichen wässrigen Zusammensetzungen eingesetzt. Ausserdem sind in der europäischen Patentanmeldungen 0 292 219 photoempfindliche und mit Säureanhydriden modifizierte Systeme beschrieben, welche Bisphenol-A-Epoxyverbindungen enthalten, deren endständige Hydroxylgruppen mit Acrylsäure modifiziert sind.

In der JP-A-5032746 werden Epoxyacrylate aus sogenannten "postglycidylisierten" Epoxyharzen (PGEH) ausschliesslich als Zwischenprodukte zur Herstellung carboxylgruppenhaltiger und vemetzbarer Komponenten für wässrig-alkalisch entwickelbare Resistformulierungen beschrieben. Andere Verwendungsmöglichkeiten sind nicht angesprochen.

Es wurde nun gefunden, dass sich höhermolekulare Epoxyacrylate aus sogenannten "postglycidylisierten" Epoxyharzen (PGEH) und zum Beispiel (Meth)acrylsäure hervorragend als Acrylatkomponente in Resistformulierungen eignen, so dass man sogar ohne oder nur mit geringen Mengen von zusätzlichen Binderpolymeren (Bindemittelpolymeren) auskommen kann, und sich zudem verbesserte thermische, mechanische, elektrische und chemische Eigenschaften ergeben.

Gegenstand der Erfindung ist die Verwendung von Epoxyacrylaten der Formel III, 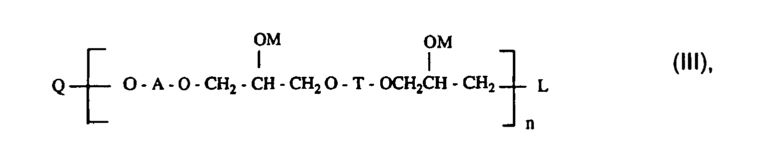 worin
- Q: Wasserstoff oder eine Gruppe der Formeln 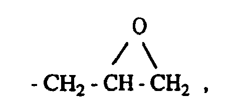 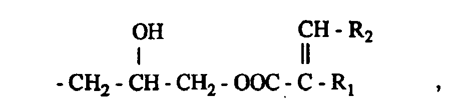 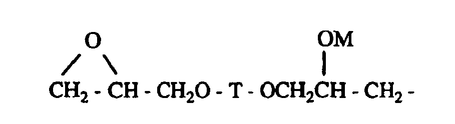 oder 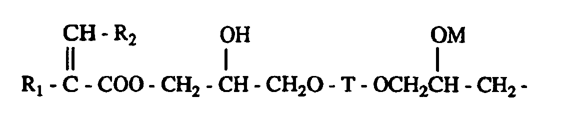 bedeutet,
- R₁: -H oder -CH₃, R₂ -H, -CH₃ oder Phenyl,
- T: den Rest einer aromatischen bifunktionellen Verbindung, und
- M: unabhängig voneinander Wasserstoff oder eine Gruppe der Formeln 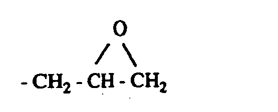 oder 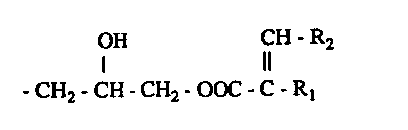 bedeuten, wobei
R₁ und R₂ die oben angegebene Bedeutung haben,
A den Rest einer aromatischen bifunktionellen Verbindung,
n eine ganze Zahl von 0 bis 300, und
L eine Gruppe der Formeln 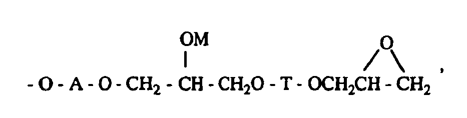 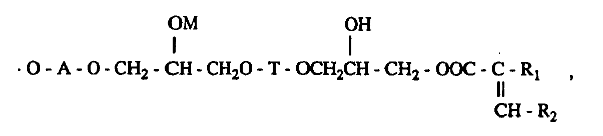
oder - O - A - OM bedeuten,
wobei in der Formel III nicht alle Reste M gleichzeitig Wasserstoff oder eine Gruppe der Formel 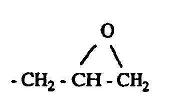 bedeuten können, sondern mindestens 10 Mol %, vorzugsweise 20-100 Mol % der Reste M, die nicht in den Endgruppen Q und L sind, eine Gruppe der Formel 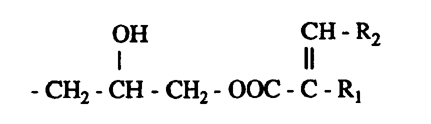 darstellen, als Acrylatkomponenten in Resistformulierungen.

Die Epoxyacrylate der Formel III werden dadurch erhalten, dass ein postglycidylisiertes Epoxyharz der Formel II 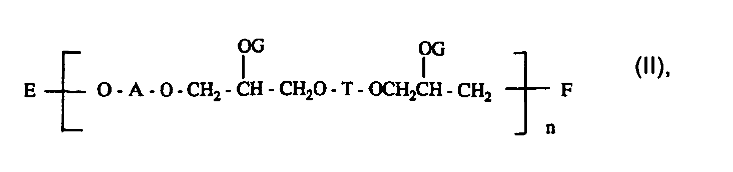 worin
- E: Wasserstoff oder eine Gruppe der Formeln 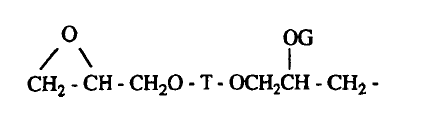 oder 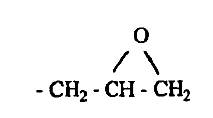
- F: die Gruppen der Formeln - O - A - OG oder 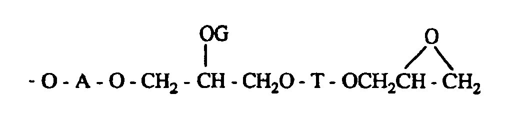 und
- G: -H oder der Rest 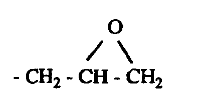 bedeuten, und
wobei analog zur Formel III mindestens 10 Mol % der Reste G in der Formel II, die nicht in den Endgruppen E und F sind, die Gruppe der Formel 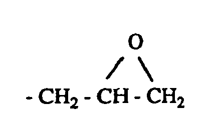 bedeuten, und
A, T, und n die angegebene Bedeutung haben,
mit einer ethylenisch ungesättigten Monocarbonsäure in Anwesenheit eines Katalysators und eines Polymerisationsinhibitors bei erhöhter Temperatur umgesetzt wird.

Für den Fall, dass n in der Formel III Null ist, bedeuten Q -H und L die Gruppe der Formel

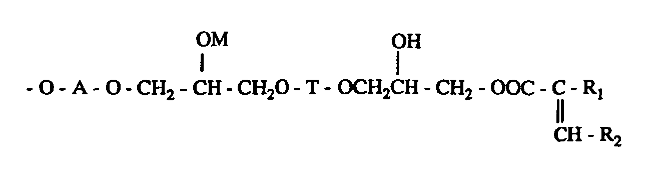

In bevorzugten Epoxyacrylaten der Formel III bedeutet n eine ganze Zahl von 0 bis 50, vor allem von 0 bis 30, und die Symbole A und T haben die in der japanischen Patentanmeldung Hei 1-195056 bevorzugten Bedeutungen von A und B.

Bevorzugte bifunktionelle aromatische Verbindungen A und T sind Brückenglieder der Formeln 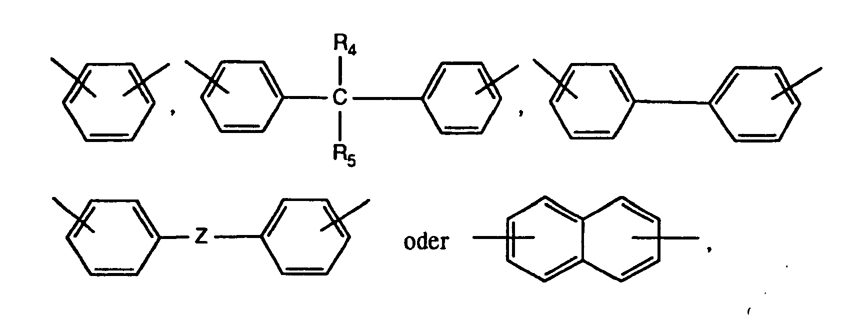 wobei R₄ und R₅ unabhängig voneinander -H oder C₁-C₄-Alkyl, Z -S-, -O- oder -SO₂ sind, und die aromatischen Reste des Brückengliedes A oder T unsubstituiert oder durch Halogen oder C₁-C₄-Alkyl substituiert sind. C₁-C₄-Alkyl ist besonders -CH₃ und Halogen bedeutet insbesondere Brom.

Besonders bevorzugte Brückenglieder A und T entsprechen unabhängig voneinander der Formel 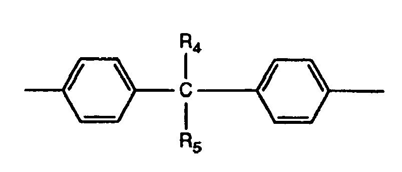 worin R₄ und R₅ die oben angegebene Bedeutung haben, und die Phenylreste des Brückengliedes unsubstituiert oder durch Brom substituiert sind, und vor allem den Formeln

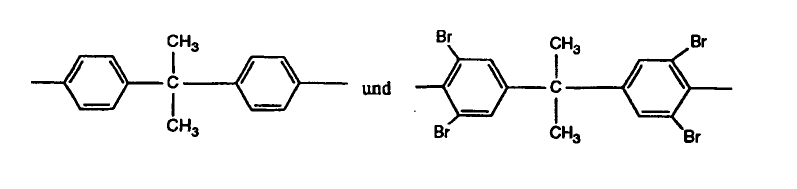

Als Epoxyacrylate werden hier und im folgenden Umsetzungsprodukte aus Epoxyverbindungen mit (Meth)acrylsäure bezeichnet.

Die postglycidylisierten Epoxyharze der Formel II sind zum Teil bekannt und werden aus den entsprechenden bekannten avancierten Epoxyharzen der Formel I 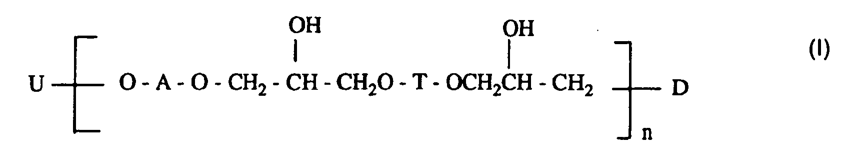 durch eine Glycidylisierungsreaktion hergestellt, worin in der Formel I
U Wasserstoff oder die Gruppe der Formel 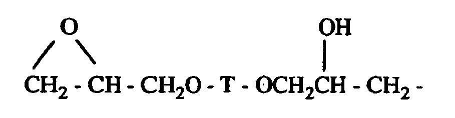 und
D die Gruppe der Formel 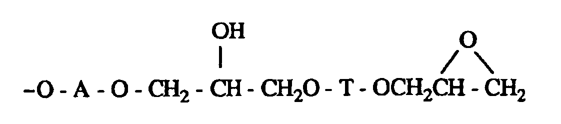 oder den Rest -O-A-OH bedeuten, wobei die Symbole A, T und n die unter der Formel III angegebene Bedeutungen haben.

Die avancierten Epoxyharze der Formel andererseits erhält man durch bekannte Polyaddition eines Bisphenols der Formel

HO-A-OH

mit einer Bisepoxyverbindung der Formel 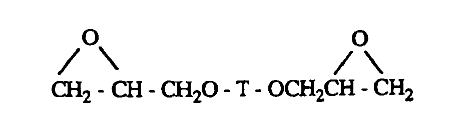 worin A und T den Rest einer bifunkdonellen aromatischen Verbindung bedeuten.

Bei den Bisphenolen HO-A-OH oder HO-T-OH handelt es sich bevorzugt um bekannte Bisphenole, vor allem um Bisphenol A und Tetrabrom-Bisphenol A, sowie um solche, die in der japanischen Patentanmeldung Hei 1-195056 beschrieben sind, insbesondere Bisphenol A, Bisphenol F, Tetrabrombisphenol A und Tetrabrombisphenol F.

Normalerweise werden zur Herstellung der avancierten Epoxyharze die oben angegebenen Bisepoxyverbindungen im Ueberschuss eingesetzt, so dass die avancierten Epoxyharze der Formel I Epoxyendgruppen aufweisen. Es ist aber auch möglich, das Bisphenol HO-A-OH im Überschuss einzusetzen und damit Moleküle mit phenolischen Endgruppen herzustellen. Das Molekulargewicht wird durch das molare Verhältnis von Bisphenol HO-A-OH zur Bisepoxyverbindung bestimmt. Gegebenenfalls können auch kleine Mengen an höherfunktionellen Phenolen oder Epoxyverbindungen (z.B. Trisphenole oder Trisepoxyphenylverbindungen) der Polyaddition zugesetzt werden. Es können auch gewisse Mengen der Ausgangsprodukte (Bisphenol HO-A-OH und/oder Bisepoxyverbindung) im avancierten Epoxyharz vorhanden sein.

Die avancierten Epoxyharze der Formel I weisen sekundäre, aliphatische Hydroxylgruppen auf, die sich aus der Additionsreaktion der phenolischen Hydroxylgruppe mit dem Oxiranring 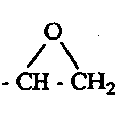 ergeben.

Die Glycidylisierungsreaktion zu den postglycidylisierten Epoxyharzen der Formel II erfolgt nach bekannten Methoden, indem das avancierte Epoxyharz (I) mit z.B. Epichlorhydrin im Überschuss in Gegenwart einer Base (z.B. NaOH) und eines Katalysators bei erhöhter Temperatur zur Reaktion gebracht wird.

Die Menge der eingesetzten Base in der Glycidylisierungsreaktion richtet sich nach dem gewünschten Glycidylisierungsgrad; vorzugsweise werden 0,1 bis 1,2 Äquivalente Base pro Äquivalent sekundare HO-Gruppe im avanzierten Epoxyharz verwendet. Wasser wird durch azeotrope Destillation mit überschüssigem Epichlorhydrin als Schleppmittel entfernt.

Als Katalysatoren kommen vor allem quaternäre Ammoniumsalze oder Phosphoniumsalze, wie Tetramethylammoniumchlorid, Benzyltrimethylammoniumchlorid, Benzyltriethylammoniumchlorid, Tetraethyl- und Tetrabutylammoniumbromid, in Frage.

Die Reaktionstemperatur liegt zweckmässig etwa zwischen 40 bis 80°C, vorzugsweise zwischen 50 und 65 °C.

Durch die Glycidylisierungsreaktion werden die aliphatischen OH-Gruppen teilweise oder vollständig glycidylisiert.

Die weitere Umsetzung der postglycidylisierten Epoxidharze der Formel II zu den Epoxyacrylaten der Formel III erfolgt ebenfalls auf bekannte Art und Weise, durch Reaktion mit einer ethylenisch ungesättigten Monocarbonsäure der Formel

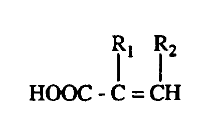

Es kommen z.B. Crotonsäure, Zimtsäure und vor allem Acrylsäure oder Methacrylsäure oder deren Mischung in Betracht. R₁ und R₂ haben die oben angegebene Bedeutung.

Bei der Reaktion wird bevorzugt ein Katalysator eingesetzt. Als Katalysatoren kommen vor allem Metallsalze, wie z.B. Chrom Verbindungen, Amine, wie Triethylamin oder Benzyldimethylamin, ferner Ammoniumsalze, wie z.B. Benzyltrimethylammoniumchlorid, oder dann auch Triphenylphosphin und Triphenylwismuth, in Frage.

Gegebenenfalls wird der Reaktion ein Lösungsmittel zugefügt, da die postglycidylisierten Epoxyharze der Formel II als Feststoffe vorliegen. Das Lösungsmittel muss jedoch gegenüber dem Edukt inert sein. Als Lösungsmittel kommen z.B. in Frage: Ketone, wie Aceton, Methylethylketon, Cyclohexanon; Ester wie Essigsäureethyl- und -butylester, Ethoxyethylacetat oder Methoxypropylacetat; Ether, wie Dimethoxyethan und Dioxan; aromatische Kohlenwasserstoffe, wie Toluol, Benzol und Xylole, sowie Gemische von zwei oder mehreren dieser genannten Lösungsmittel.

Die Temperatur variiert zweckmässig zwischen 80 und 140°C, wobei die Reaktion mit Acrylsäure bevorzugt bei 80 bis 120°C und mit Methacrylsäure bevorzugt bei 80 bis 140°C durchgeführt wird.

Gegebenenfalls kann dem Reaktionsmedium auch ein Polymerisationsinhibitor zugesetzt werden; als solche kommen z.B. in Frage: Hydrochinon, Hydrochinonmonomethylether und 2,6-Di-tert.-butyl-p-kresol.

Es ist zweckmässig, in das Reaktionsmedium Luft oder ein Gemisch aus Stickstoff/Sauerstoff einzuleiten, da einige der oben genannten Polymerisationsinhibitoren nur in Anwesenheit von Sauerstoff wirksam sind. In Abhängigkeit der eingesetzten Menge der ethylenisch ungesättigten Monocarbonsäure werden Epoxyacrylate der Formel III erhalten, die vollständig oder nur teilweise acryliert sind. Dabei kann die Monocarbonsäure in äquimolaren Mengen bezüglich der Epoxygruppen oder im Unterschuss eingesetzt werden. Die vollständig umgesetzten Epoxyacrylate enthalten praktisch keine Epoxygruppen mehr.

Die Epoxyacrylate der Formel III müssen weder aus dem Reaktionsmedium isoliert noch gereinigt werden. Die erhaltene Reaktionslösung kann als solche verwendet werden.

Sowohl die teilweise als auch die vollständig umgesetzten Produkte der Formel III enthalten aliphatische Hydroxylgruppen, die aus der Reaktion der Epoxygruppen mit der ethylenisch ungesättigten Monocarbonsäure herrühren. Zusätzlich können noch aliphatische Hydroxylgruppen aus dem Edukt vorhanden sein.

Infolge der im Molekül vorhandenen ungesättigten Gruppen sind die Epoxyacrylate der Formel III thermisch und photochemisch vernetzbar. Sie können deshalb als Acrylatkomponenten zum Beispiel in Photoresistformulierungen für die Herstellung von Lötstoppresists oder Primärresists nach bekannten Verfahren verwendet und appliziert werden, wie z.B. in der am 2. Juli 1993 hinterlegten Schweiz. Patentanmeldung Nr. 2005/93-4 "Photopolymerisierbare Zusammensetzungen", und ergeben Resistschichten mit verbesserten thermischen, mechanischen, elektrischen und chemischen Eigenschaften. Die daraus hergestellten Resistformulierungen werden vor allem auf dem Gebiet der Leiterplatten z.B. als Lötstoppresist oder Primärresist, und der Druckplatten angewandt. Des weiteren kommen sie zur Herstellung von Offsetdruckplatten, Flexodruckplatten, Buchdruckplatten und Siebdruckformulierungen in Frage. Als Entwickler kommen sowohl wässrige als auch wässrig/organische oder organische Systeme in Frage.

Gegenüber niedermolekularen Epoxyacrylaten in Formulierungen, die Binderpolymere enthalten, ist es überraschend, dass Formulierungen mit höhermolekularen Epoxyacrylaten ohne Zusatz derartiger Binderpolymere keinen Verlust an sondern eine Verbesserung der Photoempfindlichkeit aufweisen, und auch dass keine Erhöhung der Klebrigkeit erfolgt. Des weiteren ist eine bessere Kantendeckung der Leiterzüge bei ihrer Verwendung als Lötstoppresist gegeben. Da in derartigen Formulierungen keine zusätzlichen Binderpolymere eingesetzt werden, können weitere Vorteile bezüglich der thermischen, mechanischen und elektrischen Eigenschaften und insbesondere bezüglich der chemischen Beständigkeit der daraus gewonnenen Resistzusammensetzungen erhalten werden. Ferner weisen die Epoxyacrylate der Formel III eine erhöhte Glasumwandlungstemperatur auf.

Die folgenden Beispiele veranschaulichen die Erfindung, ohne sie darauf zu limitieren.

### A) Herstellung von postalycidylisierten Epoxyharzen (PGEH)

Beispiel 1: Es wird eine für Tetramethylammoniumchlorid (TMAC)-Glycidylisierungen geeignete Apparatur verwendet, die es gestattet, überschüssiges Epichlorhydrin als Schleppmittel für die Wasserabscheidung unter vermindertem Druck einzusetzen. Es handelt sich dabei um ein 5000 ml Reaktionsgefäss mit Rührer, Thermometer, einem gut wirksamen Intensivkühler und 2 Tropftrichtern mit Druckausgleich. Angeschlossen sind ein Wasserabscheider (für obenliegende wässrige Phase und Entleerung unter Vakuum) und eine Wasserstrahlpumpe mit Manometer. Die Heizung geschieht mittels Ölbad.

1000 g Epoxidharz Araldit GT 7004 der Firma CIBA (Epoxygruppengehalt: 1,36 Mol/kg Harz; HO-Gruppen-Gehalt: 2,71 Mol) und 1700 ml (21,68 Mol) Epichlorhydrin werden im Reaktor durch Erwärmen gelöst. Die erhaltene homogene Lösung wird auf eine Innentemperatur von ca. 80°C erwärmt, und unter guter Rührung wird das Wasserstrahlvakuum so vorsichtig angelegt, dass das Epichlorhydrin kräftig am Rückfluss siedet. Die Innentemperatur sinkt ab und wird auf einen konstanten Wert von ca. 55 °C eingestellt und während der gesamten Reaktion gehalten. Dies bedingt anfänglich einen Druck von 110-120 mbar, den man gegen Schluss hin eventuell etwas senken muss. Die Ölbadtemperatur beträgt konstant 105-110°C. Wenn das Epichlorhydrin bei 55°C konstant rückflussiert, tropft man aus dem ersten Tropftrichter 29,80 g (0,136 Mol) einer 50%-igen wässrigen Tetramethylammoniumchloridlösung rasch zu. Dann werden innnerhalb von 2 Stunden aus dem zweiten Tropftrichter 195,20 g (2,44 Mol) einer 50%-igen wässrgen Natriumhydroxydiösung zugetropft, und gleichzeitig werden das entstehende Wasser sowie das aus den 50%-Lösungen durch azeotrope Destillation stammende Epichlorhydrin ausgekreist. Die Innentemperatur hält man bei 55-58°C und sorgt durch genügend Rückfluss für eine zügige Entfernung des Wassers aus dem Reaktionsgemisch. Man lässt nach dem Zutropfen noch 2 Stunden bei 55°C unter Wasserauskreisung nachreagieren. Das Vakuum wird abgestellt, und die Suspension wird durch Zugabe von zerkleinertem Trockeneis, gefolgt von 10-20 ml Eisessig, neutralisiert. Nach dem Zufügen von 2000 ml Methoxypropylacetat werden das Lösungsmittel und das Epichlorhydrin unter vermindertem Druck abdestilliert. Zum Rückstand gibt man nochmals 2000 ml Methoxypropylacetat und filtriert dann die erhaltene Suspension über ein Filtrationshilfsmittel (Hyflo), wobei man von Zeit zu Zeit die Oberfläche des Filterbetts aufkratzt. Das klare, gelbliche Filtrat engt man am Rotationsverdampfer bei einer Badetemperatur von bis zu 120°C ein. Das gewonnene gelbe, klare Harz wird durch Zugabe von Methoxypropylacetat zu einer Lösung mit 50% Festkörpergehalt verdünnt. Man erhält 2316 g einer 50%-igen, praktisch farblosen Lösung des postglycidylisierten Epoxyharzes der Formel II, worin
- A: 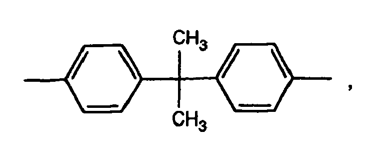
- T: 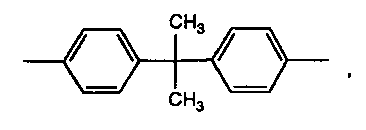
- E: 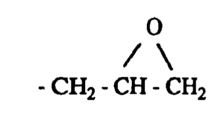 und
- F: 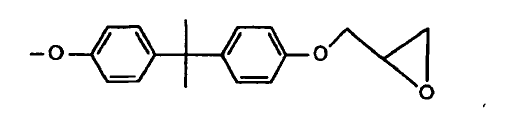 bedeuten, und
- G: etwa 90 Mol% der Gruppe 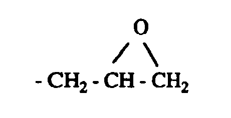 und 10 Mol% -H bedeutet, und
- n: einen Mittelwert 2 hat.

Diese Lösung kann direkt für die Umsetzung mit Acrylsäure eingesetzt werden. Analytische Daten dieser Lösung:

| | | |
|---|---|---|
| 1. | Gehaltsbestimmung (Trockengewicht): | 49,5%; |
| 2. | Epoxywert (titrimetrisch bestimmt): | 1,46 Mol/kg/(- 2,81 Mol/kg Festharz); |
| 3. | Chlorgehalt (Festharz): | 0,40% Chlor total; |
| | | 0,23% Chlor hydrolysierbar; |
| 4. | GPC (Gelpermeationschromatographie Polystyrol-Eichung | M_{w} = 10678; Mₙ = 2138. |

Falls das Festharz gewünscht wird, kann man bei der Aufarbeitung anstelle von Methoxypropylacetat als Lösungsmittel Methylisobutylketon zum Verdünnen einsetzen und den Rückstand nach dem Abrotieren (Einengen im Rotationsverdampfer) heiss in flache Stahlwannen giessen und im Hochvakuum bei 150°C mehrere Stunden trocknen.

Beispiel 2: Gemäss dem Verfahren des Beispiels 1 werden 555 g Araldit GT 7004 der Firma CIBA (Epoxygruppengehalt: 1,36 Mol/kg Harz; HO-Gruppengehalt: 1,50 Mol), 705 ml Epichlorhydrin (9,00 Mol), 16,40 g einer 50%-igen wässrigen Tetramethylammoniumchloridlösung und 66,00 g einer 50%-igen wässrigen Natriumhydroxydiösung (0,83 Mol) umgesetzt. Nach dem Zutropfen der Natronlauge lässt man noch 45 Min. unter Wasserabscheidung nachreagieren und destilliert dann bei einem Vakuum von ca. 85 mbar den Grossteil des Epichlorhydrins ab. Man fügt 600 ml Methylisobutylketon zu, neutralisiert mit Trockeneis und Essigsäure und verdünnt mit weiteren 500 ml Methylisobutylketon. Die Suspension wird über ein Filterhilfsmittel (Hyflo) filtriert, das Lösungsmittel wird abrotiert, der Rückstand wird heiss in Stahlwannen gegossen und am Hochvakuum bei 150°C mehrere Stunden getrocknet. Analytische Daten des erhaltenen leicht gelblichen Festharzes (473 g):

| | | |
|---|---|---|
| 1. | Epoxywert(titrimetrisch): | 2,39 Mol/kg Harz; |
| 2. | Chlorgehalt | 0,33% Chlor total; |
| | | 0,17% Chlor hydrolysierbar; |
| 3. | OH-Gruppengehalt(titrimetrisch): | 1,22 Mol/kg; |
| 4. | GPC (Polystyrol-Eichung): | M_{w} = 10721; Mₙ= 2330. |

Das Festharz entspricht der Formel II, worin A und T sowie E, F und n die im Beispiel 1 angegebene Bedeutungen haben und
- G: etwa 55 Mol% der Gruppe 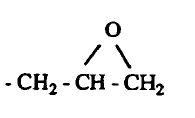 und 45 Mol% -H bedeutet.

Beispiel 3: Nach dem Verfahren gemäss Beispiel 1 werden 525 g Araldit B 41 der Firma CIBA (Epoxygruppengehalt: 2,66 Mol/kg Harz; HO-Gruppengehalt: 1,01 Mol), 712 ml Epichlorhydrin (9,09 Mol), 18,48 g einer 50%-igen wässrigen Tetramethylammoniumchloridlösung und 89,00 g einer 50%-igen wässrigen Natriumhydroxydiösung (1,11 Mol) umgesetzt und als Festharz isoliert.

Analytische Daten des praktisch farblosen Festharzes (515 g):

| | | |
|---|---|---|
| 1. | Epoxywert (titrimetrisch): | 3,48 Mol/kg; |
| 2. | Chlorgehalt: | 0,21 % Chlor total; |
| | | <0,05% Chlor hydrolysierbar; |
| 3. | GPC (Polystyrol-Eichung): | M_{w} = 2374; Mₙ = 935. |

Das Festharz entspricht der Formel II, worin T, E und F die im Beispiel 1 angegebene Bedeutungen haben, n einen Mittelwert von etwa 0,7 aufweist und G 100Mol% 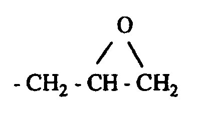 bedeutet.

Beispiel 4: Nach dem Verfahren gemäss Beispiel 1 werden 435 g Araldit GT 6071 der Firma CIBA (Epoxygruppengehalt: 2,18 Mol/kg Harz; HO-Gruppengehalt: 1,00 Mol), 470 ml Epichlorhydrin (6,00 Mol), 11,00 g einer 50%-igen wässrigen Tetramethylammoniumchloridlösung und 88,00 g einer 50%-igen wässrigen Natriumhydroxydiösung (1,10 Mol) umgesetzt und als Festharz isoliert.

Analytische Daten des leicht gelblichen Festharzes (390 g):

| | | |
|---|---|---|
| 1. | Epoxywert (titrimetrisch): | 3,55 Mol/kg; |
| 2. | Chlorgehalt: | 0,82% Chlor total; |
| | | 0,60% Chlor hydrolysierbar; |
| 3. | GPC (Polystyrol-Eichung): | M_{w} = 6498; Mₙ= 1376. |

Das Festharz entspricht der Formel II, worin A, T, E und F die im Beispiel 1 angegebene Bedeutungen haben und n einen Mittelwert von 1,0 aufweist und G 100 Mol% 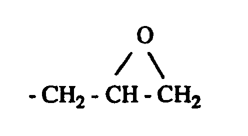 bedeutet,

Beispiel 5: Nach dem Verfahren gemäss Beispiel 1 werden 1000 g Araldit GT 6097 der Firma CIBA (Epoxygruppengehalt: 0,60 Mol/kg Harz; HO-Gruppengehalt: 3,175 Mol), 1996 ml Epichlorhydrin (25,40 Mol), 35,20 g einer 50%-igen wässrigen Tetramethylammoniumchloridlösung und 228,60 g einer 50%-igen wässrigen Natriumhydroxydiösung (2,86 Mol) umgesetzt und als ca. 50%-Lösung in Methoxypropylacetat isoliert.

Analytische Daten dieser Lösung (2283 g):

| | | |
|---|---|---|
| 1. | Festkörpergehalt: | 1.48,30%; |
| 2. | Epoxywert (titrimetrisch): | 1,17 Mo/kg; |
| 3. | Chlorgehalt: | 0,63% Chlor total; |
| | | 0,30% Chlor hydrolysierbar; |
| 4. | GPC (Polystyrol-Eichung): | M_{w} = 16759; Mn = 3382. |

Der Festkörper in der Lösung entspricht der Formel II, worin A, T, E und F die im Beispiel 1 angegebene Bedeutungen haben, n einen Mittelwert von 5,3 aufweist und
G 90 Mol% 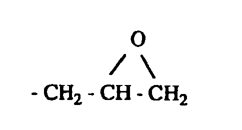 und 10 Mol% -H bedeutet.

Beispiel 6: Nach dem Verfahren gemäss Beispiel 1 werden 500 g Araldit GT 7097 der Firma CIBA (Epoxygruppengehalt: 0,58 Mol/kg Harz; HO-Gruppengehalt: 1,60 Mol), 753 ml Epichlorhydrin (9,60 Mol), 17,54 g einer 50%-igen wässrigen Tetramethylammoniumchloridlösung und 115,20 g einer 50%-igen wässrigen Natriumhydroxydiösung (1,44 Mol) umgesetzt und als ca. 45%-Lösung in Methoxypropylacetat isoliert.

Analytische Daten dieser Lösung (1200 g):

| | | |
|---|---|---|
| 1. | Festkörpergehalt: | 44,50%; |
| 2. | Epoxywert (titrimetrisch): | 1, 19 Mol/kg; |
| 3. | Chlorgehalt: | 0,70% Chlor total; |
| | | 0,45% Chlor hydrolysierbar; |
| 4. | GPC (Polystyrol-Eichung): | M_{w} = 23091; Mₙ = 4270. |

Der Festkörper in der Lösung entspricht der Formel II, worin A, T, E und F die im Beispiel 1 angegebenen Bedeutungen haben, n einen Mittelwert von 5,5 aufweist und G 90Mol% 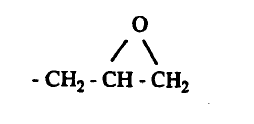 und 10 Mol% -H bedeutet.

Beispiel 7: Nach dem Verfahren gemäss Beispiel 1 werden 400 g Araldit GT 6099 der Firma CIBA (Epoxygruppengehalt: 0,41 Mol/kg Harz; HO-Gruppengehalt: 1,32 Mol), 1038 ml Epichlorhydrin (11,88 Mol), 14,08 g einer 50%-igen wässrigen Tetramethylammoniumchloridlösung und 95,04 g einer 50%-igen wässrigen Natriumhydroxydiösung (1,19 Mol) umgesetzt und als ca. 45%-Lösung in Methoxypropylacetat isoliert.

Analytische Daten dieser Lösung (915 g):

| | | |
|---|---|---|
| 1. | Festkörpergehalt: | 45,60%; |
| 2. | Epoxywert (titrimetrisch): | 1, 16 Mol/kg; |
| 3. | Chlorgehalt: | 0,42% Chlor total; |
| | | 0,24% Chlor hydrolysierbar; |
| 4. | GPC (Polystyrol-Eichung): | M_{w} = 57017; Mₙ = 5604. |

Der Festkörper in der Lösung entspricht der Formel II, worin A, T, E und F die in Beispiel 1 angegebenen Bedeutungen haben,
n etwa 8,0 ist und
G 90Mol% 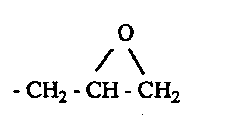 und 10 Mol% -H bedeutet.

Beispiel 8: Nach dem Verfahren gemäss Beispiel 1 werden 1171 g Araldit Festharz der Firma CIBA (Bromhaltiges Epoxyharz; Epoxy gruppengehalt: 1,85 Mol/kg Harz; HO-Gruppengehalt: 2,26 Mol), 1416 ml Epichlorhydrin (18,10 Mol), 24,80 g einer 50%-igen wässrigen Tetramethylammoniumchloridlösung und 162,60 g einer 50%-igen wässrigen Natriumhydroxydiösung (2,03 Mol) umgesetzt und als Festharz isoliert.

Analytische Daten dieses Festharzes (1050 g):

| | | |
|---|---|---|
| 1. | Epoxywert (titrimetrisch): | 2,40 Mol/kg; |
| 2. | Chlorgehalt: | 0,45% Chlor total; |
| | | 0,22% Chlor hydrolysierbar; |
| 3. | GPC (Polystyrol-Eichung): | M_{w} = 3189; Mₙ = 1235. |

Das Festharz entspricht der Formel II, worin
A 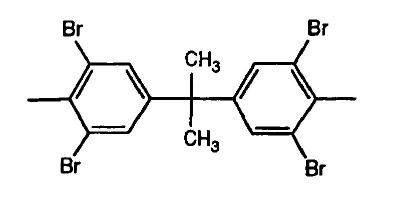 und
T 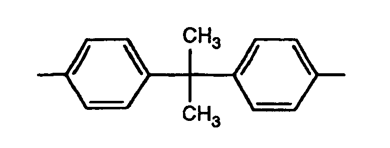 bedeuten, und
E und F die im Beispiel 1 angegebenen Bedeutungen haben,
n etwa 1,0 ist und
G 90 Mol% 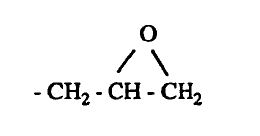 und 10 Mol% -H bedeutet.

### B) Teilweise oder vollständig acrylierte PGEH Epoxyacrylate

Beispiel 9: Als Apparatur verwendet man ein 5000 ml Reaktionsgefäss, ausgestattet mit Rührer, Tropftrichter, Thermometer, Rückflusskühler und einem Einleitungsrohr für Luft. Zur Inhibierung der Polymerisation der Acrylate wird während der Reaktion ein schwacher Luftstrom unter Niveau eingeleitet. Die Heizung geschieht mittels thermostatisierbarem Ölbad. Im Reaktor werden 2316,5 g einer 45%-igen Lösung (in Methoxypropylacetat) des Reaktionsproduktes gemäss Beispiel 1 (2,965 Mol Epoxygruppen), 3,80 g Hydrochinonmonomethylether und 5,20 ml Triethylamin vorgelegt und unter Rühren auf 95°C Innentemperatur aufgeheizt. Unter Einleitung eines schwachen Luftstromes dosiert man 213,70 g Acrylsäure (2,965 Mol) innerhalb einer Stunde bei einer Innentemperatur von 95°C zu und lässt 25 Stunden bei dieser Temperatur weiter reagieren. Die Reaktion wird durch Messen des Epoxywertes verfolgt; am Schluss beträgt er weniger als 0,08 Mol/kg. Das Reaktionsprodukt wird abgekühlt und ohne zusätzliche Reinigung weiter verwendet.

| | | |
|---|---|---|
| 1. | Festkörpergehalt: | 54%; |
| 2. | Säuregehalt (titrimetrisch): | 0,03 Mol/kg ; |
| 3. | Viskosität (Brookfield) 25°C: | 1970 mPa.s; |
| 4. | GPC (Polystyrol-Eichung): | M_{w} = 8889; Mₙ = 2256. |

Der Festkörper entspricht der Formel III, worin A und T die im Beispiel 1 angegebenen Bedeutungen haben,
Q 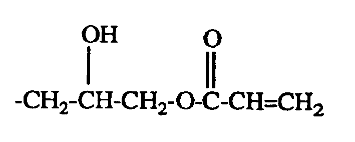 und
L 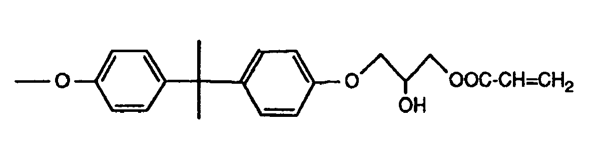 bedeuten, und
n einen Wert von etwa 2,0 hat und
M 90 Mol% 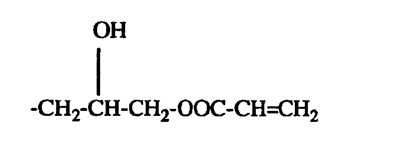 und 10 Mol% -H bedeutet.

Beispiel 10: Als Apparatur verwendet man ein 2000 ml Reaktionsgefäss, ausgestattet mit Rührer, Thermometer, Rückflusskühler und einem Einleitungsrohr für Luft. Zur Inhibierung der Polymerisation der Acrylate wird während der Reaktion ein schwacher Luftstrom unter Niveau eingeleitet. Die Heizung geschieht mittels thermostatisierbarem Ölbad. Der Reaktor wird mit folgenden Komponenten beschickt und bei Raumtemperatur bis zum Erreichen der homogenen Lösung gerührt: 1018,60 g einer 50%-igen Lösung (in Methoxypropylacetat) des Reaktionsproduktes gemäss Beispiel 1 (1,422 Mol Epoxygruppen titriert); 102,46 g Acrylsäure (1,422 Mol); 1,53 g Hydrochinonmonomethylether; 20,38 ml einer 10%-igen Lösung (in Methoxypropylacetat) von Nuosynchromium 5 (Produkt der Firma HARCROS - Durham Chemicals, Durham DH3 1QX, GB). Das Reaktionsgemisch wird auf eine Innentemperatur von 110°C aufgeheizt und bei dieser Temperatur unter Rühren und Einleiten eines schwachen Luftstroms während 7 Stunden gehalten. Das Fortschreiten der Reaktion lässt sich mittels Säure- oder Epoxytitration verfolgen. Nach 7 Stunden ergab die Säuretitration einen Wert von 0,05 Mol/kg. Das Reaktionsprodukt wird nach dem Abkühlen abgefüllt und ohne weitere Reinigung weiter verwendet.

Analytische Daten des Reaktionsproduktes (1080 g):

| | | |
|---|---|---|
| 1. | Festkörpergehalt: | 53,3%; |
| 2. | Epoxywert (titrimetrisch): | 0,02 Mol/kg ; |
| 3. | Viskosität (Brookfield) 25°C: | 1970 mPa.s; |
| 4. | GPC (Polystyrol-Eichung): | M_{w} = 12168; Mₙ = 2602. |

entspricht der chemischen Struktur gemäss Beispiel 9.

### Beispiel 11: Apparatur (500 ml Reaktor) gemäss Beispiel 10.

Nach dem Verfahren von Beispiel 10 werden 200 g einer 50%-igen Lösung (in Methoxypropylacetat) des Reaktionsproduktes gemäss Beispiel 1 (0,292 Mol Epoxygruppen), 10,52 g Acrylsäure (0,146 Mol), 0,33 g Hydrochinonmonomethylether und 20,00 ml einer 1%-igen Lösung (in Methoxypropylacetat) von Nuosynchromium 5 umgesetzt. Die Reaktion ist nach 3 Stunden bei 100°C Innentemperatur beendet, die Säuretitration ergibt dann <0,02 Mol Säure.

Analytische Daten des Reaktionsproduktes (210 g):

| | | |
|---|---|---|
| 1. | Festkörpergehalt: | 46%; |
| 2. | Epoxywert (titrimetrisch): | 0,71 Mol/kg ; |
| 3. | GPC (Polystyrol-Eichung): | M_{w} = 11036; Mₙ = 2352. |

Der Festkörper entspricht der Formel III, worin A und T die im Beispiel 1 angegebenen Bedeutungen haben,
Q 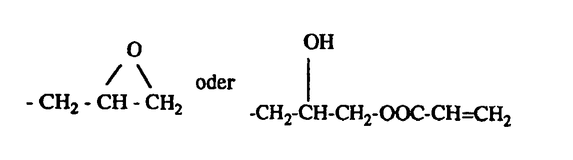 L 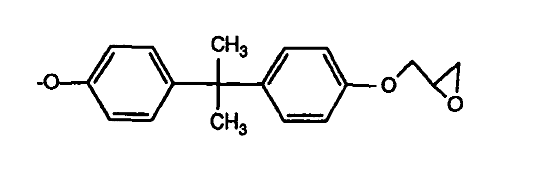 oder 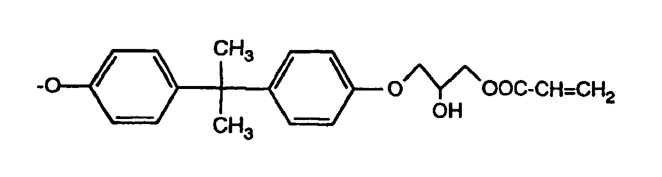 bedeuten und
n einen Wert von 2 hat, und
M 90Mol% 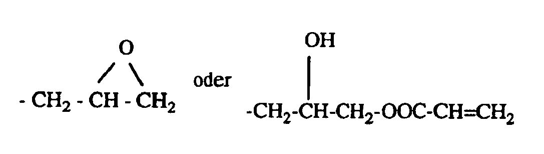 und 10 Mol% -H ist.

### Beispiel 12: Apparatur gemäss Beispiel 11.

Nach dem Verfahren von Beispiel 10 werden 200 g einer 50%-igen Lösung (in Methoxypropylacetat) des Reaktionsproduktes gemäss Beispiel 5 (0,236 Mol Epoxygruppen),17,00 g Acrylsäure (0,236 Mol), 0,33 g Hydrochinonmonomethylether und 0,54 g Triphenylphosphin umgesetzt. Die Reaktion ist nach 14 Stunden bei 100°C Innentemperatur beendet, die Säuretitration ergibt dann 0,09 Mol Säure/kg.

Analytische Daten des Reaktionsproduktes (210 g):

| | | |
|---|---|---|
| 1. | Festkörpergehalt: | 54%; |
| 2. | Epoxywert (titrimetrisch): | 0,10 Mol/kg; |
| 3. | GPC (Polystyrol-Eichung): | M_{w} = 19493; Mₙ = 3075. |

Der Festkörper entspricht der Formel III, worin A und T die im Beispiel 1 und Q, L, M die im Beispiel 9 angegebene Bedeutung haben und n einen Wert von etwa 5,3 hat.

### Beispiel 13: Apparatur gemäss Beispiel 11.

Nach dem Verfahren von Beispiel 10 werden 100 g einer 48,3%-igen Lösung (in Methoxypropylacetat) des Reaktionsproduktes gemäss Beispiel 5 (0,117 Mol Epoxygruppen), 8,85 g Acrylsäure (0,123 Mol), 0,15 g Hydrochinonmonomethylether und 1,15 ml einer 10%-igen Lösung (in Methoxypropylacetat) von Nuosynchromium 5 umgesetzt. Die Reaktion ist nach 7 Stunden bei 110°C Innentemperatur beendet, die Säuretitration ergibt dann 0,06 Mol/kg. Analytische Daten des Reaktionsproduktes (210 g):

| | |
|---|---|
| Epoxywert (titrimetrisch): | 0,020 Mol/kg. |

Das Produkt entspricht der Formel gemäss Beispiel 12.

Beispiel 14: Apparatur (1000 ml Reaktor) gemäss Beispiel 10. Nach dem Verfahren von Beispiel 10 werden 720 g einer 50%-igen Lösung (in Methoxypropylacetat) des Reaktionsproduktes gemäss Beispiel 5 (0,828 Mol Epoxygruppen); 29,83 g Acrylsäure (0,414 Mol), 1,11 g Hydrochinonmonomethylether und 37,00 ml einer 2%-igen Lösung (in Methoxypropylacetat) von Nuosynchromium 5 umgesetzt. Nach 5 Stunden bei 100°C Innentemperatur ist die Reaktion beendet, die Säuretitration ergibt dann <0,02 Mol Säure/kg.

Analytische Daten dieses Reaktionsproduktes:

| | | |
|---|---|---|
| 1. | Festkörpergehalt: | 50,3%; |
| 2. | Epoxywert (titrimetrisch): | 0,65 Mol/kg; |
| 3. | GPC (Polystyrol-Eichung): | M_{w} = 38840; Mₙ = 4226. |

Der Festkörper entspricht der Formel III, worin A und T die im Beispiel 1 und Q, L und M die im Beispiel 11 angegebenen Bedeutungen haben, und n einen Wert von etwa 5,3 hat.

### Applikationsbeispiele

Generelles: Als Beschichtungssubstrate dienen gereinigte kupferkaschierte Elektroniklaminate oder prozessierte Leiterplatten, die ein Leiterbahnmuster aufweisen. Die Resistrezepturen werden durch Zusammenmischen und Lösen der in den Beispielen aufgeführten Komponenten, eventuell gefolgt von einer Filtration, hergestellt. Alle Operationen sind unter Gelblichtschutz durchzuführen. Für Testzwecke kann man die Rezepturen mit einem Drahtrakel auf die Leiterplatte beschichten. Für grössere Serien werden vor allem Vorhanggiessverfahren oder Walzenbeschichtung sowie Siebdruckverfahren eingesetzt. Die Trocknung geschieht in einem Umluftofen. Zur Belichtung verwendet man kommerzielle Geräte mit 5000 W Metallhalogenid - dotierten Quecksilberhochdruckstrahler. Die Entwicklung wird in kommerziellen Durchlaufentwicklungsgeräten durchgeführt. Zur Beurteilung der Photoempfindlichkeit und des Auflösungsvermögens belichtet man durch Stufenkeil und Auflösungskeil der Fa. Stouffer und evaluiert das Ergebnis anhand des entwickelten Resistbildes.

## Patentansprüche

1. Verwendung von Epoxyacrylaten der Formel III, 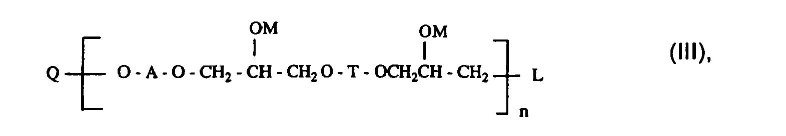 worin
Q Wasserstoff oder eine Gruppe der Formeln 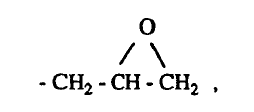 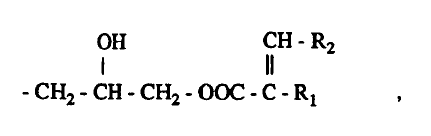 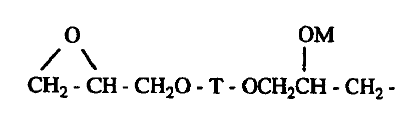 oder 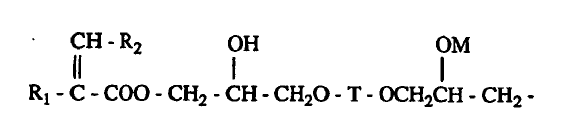 bedeutet,
R₁ -H oder -CH₃, R₂ -H, -CH₃ oder Phenyl,
T den Rest einer aromatischen bifunktionellen Verbindung, und
M unabhängig voneinander Wasserstoff oder eine Gruppe der Formeln 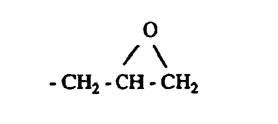 oder 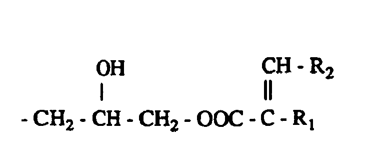 bedeuten, wobei
R₁ und R₂ die oben angegebene Bedeutung haben,
A den Rest einer aromatischen bifunktionellen Verbindung,
n eine ganze Zahl von 0 bis 300, und
L eine Gruppe der Formeln 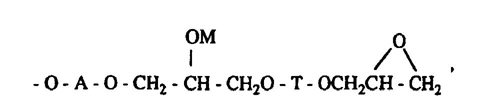 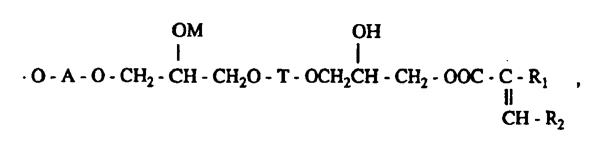
oder - O - A - OM bedeuten,
wobei in der Formel III nicht alle Reste M gleichzeitig Wasserstoff oder eine Gruppe der Formel 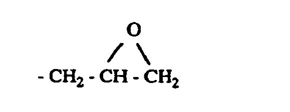 bedeuten können, sondern mindestens 10 Mol %, vorzugsweise 20-100 Mol % der Reste M, die nicht in den Endgruppen Q und L sind, eine Gruppe der Formel 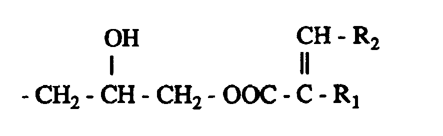 darstellen,
als Acrylatkomponenten in Resistformulierungen.

2. Verwendung gemäss Anspruch 1, worin in Formel III R₁ Wasserstoff oder Methyl und R₂ Wasserstoff, Methyl oder Phenyl bedeuten.

3. Verwendung gemäss Anspruch 1, worin in Formel III n eine ganze Zahl von 0 bis 50 bedeutet.

4. Verwendung gemäss Anspruch 1, worin n in Formel III Null ist, Q -H und L eine Gruppe der Formel 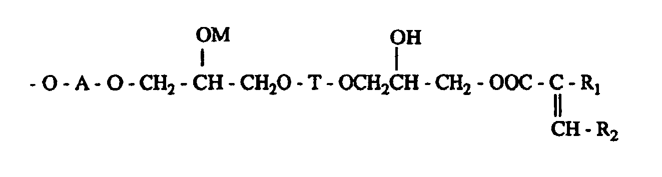 bedeuten, worin A, T, R₁ und R₂ die in Anspruch 1 angegebenen Bedeutungen haben.

5. Verwendung gemäss Anspruch 1, worin in Formel III A und T den Formeln 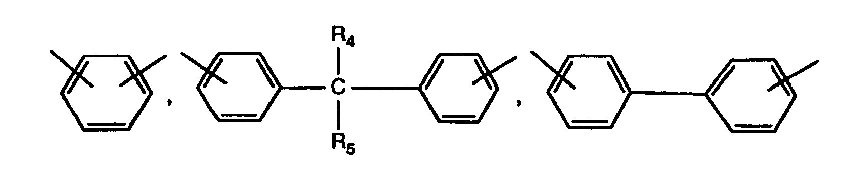 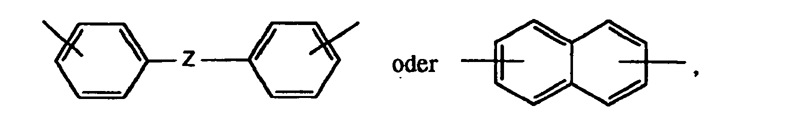 entsprechen, R₄ und R₅ unabhängig voneinander -H oder C₁-C₄-Alkyl, Z -S-, -O- oder -SO₂ sind, und die aromatischen Reste des Brückengliedes A oder T unsubstituiert oder mit Halogen oder C₁-C₄-Alkyl substituiert sind.

6. Verwendung gemäss Anspruch 5, worin R₄ und R₅ -CH₃ ist und Halogen Brom bedeutet.

7. Verwendung gemäss Anspruch 5, worin in Formel III A und T der Formel 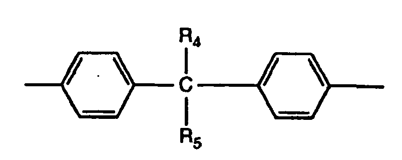 entsprechen, worin R₄ und R₅ die in Anspruch 6 angegebenen Bedeutungen haben, und die Phenylreste des Brückengliedes unsubstituiert oder mit Brom substituiert sind.

8. Verwendung gemäss Anspruch 5, worin A und T den Formeln 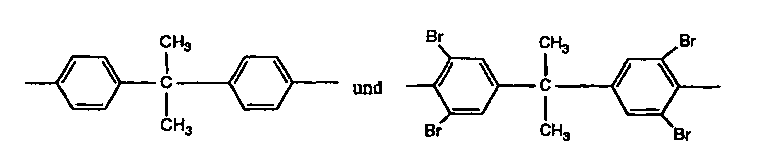 entspricht.

9. Verwendung von Epoxyacrylaten gemäss Anspruch 1 zur Herstellung von Lötstoppresists oder Primärresists.

## Claims

1. Use of epoxy acrylates of formula III, 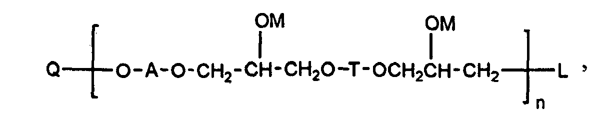 wherein
Q represents hydrogen or a group of formula 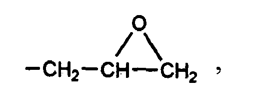 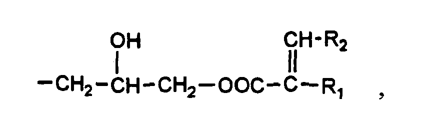 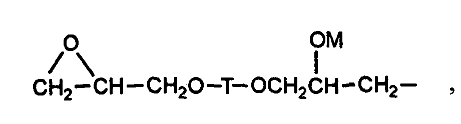 or 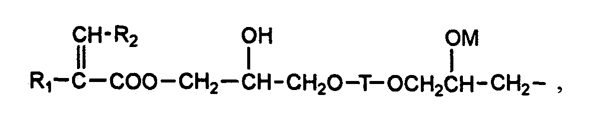
R₁ represents -H or -CH₃, R₂ represents -H, -CH₃ or phenyl,
T represents the residue of an aromatic bifunctional compound and
M represent independently from each other hydrogen or a group of the formula 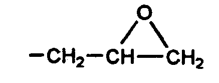 or 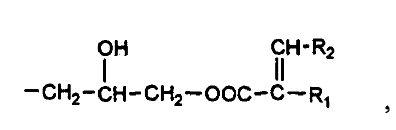 wherein
R₁ and R₂ are defined as above,
A represents the residue of an aromatic bifunctional compound,
n represents an integer from 0 to 300, and
L represents a group of the formula 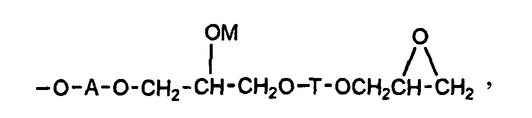 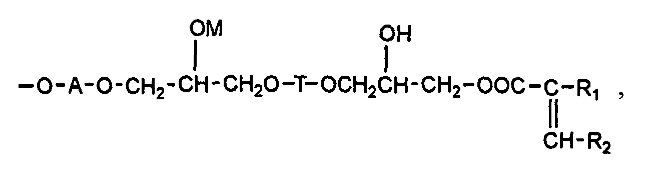
or -O-A-OM,
wherein in formula III not all moieties M can represent at the same time hydrogen or a group of formula 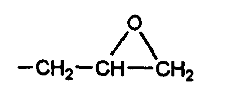 but at least 10 Mol %, preferably 20-100 Mol% of the moieties M, which are not in the end groups Q and L, represent a group of the formula 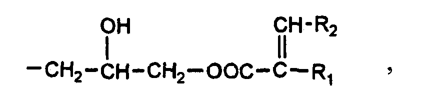 as acrylate components in resist formulations.

2. Use according to claim 1, wherein in formula III R₁ represents hydrogen or methyl and R₂ represents hydrogen, methyl or phenyl.

3. Use according to claim 1, wherein in formula III n represents an integer from 0 to 50.

4. Use according to claim 1, wherein n in formula III is zero, Q represents -H and L the group of the formula 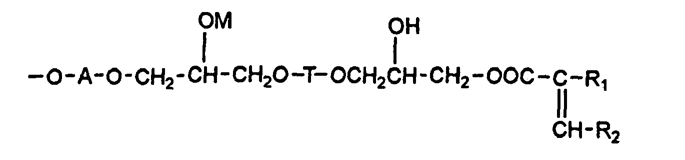 wherein A, T, R₁ and R₂ are defined as in claim 1.

5. Use according to claim 1, wherein in formula III A and T correspond to formulas 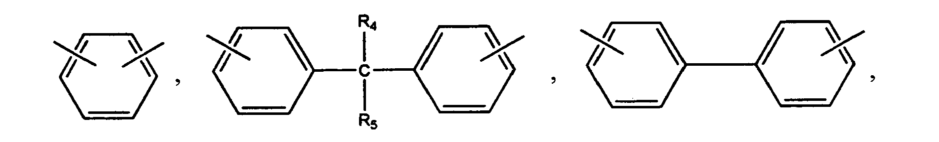 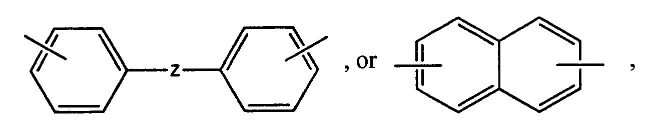 R₄ and R₅ are independently from each other -H or C₁-C₄ alkyl, Z is -S-, -O- or -SO₂, and the aromatic residues of the bridging units A or T are unsubstituted or substituted with halogen or C₁-C₄-alkyl.

6. Use according to claim 5, wherein R₄ and R₅ are -CH₃ and halogen represents bromine.

7. Use according to claim 5, wherein in formula III A and T correspond to formula 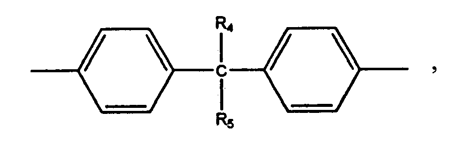 wherein R₄ and R₅ are defined as in claim 6, and the phenyl residues of the bridging unit are unsubstituted or substituted with bromine.

8. Use according to claim 5, wherein A and T correspond to formulas

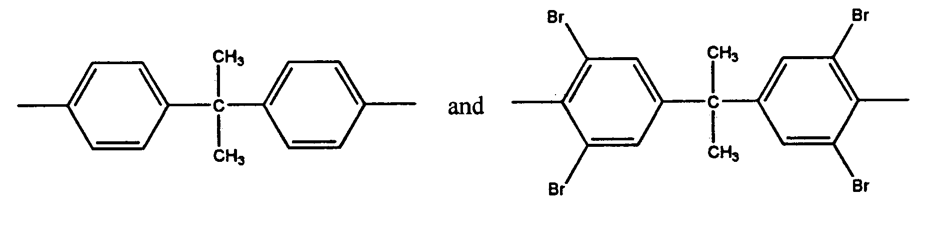

9. Use of epoxy acrylates according to claim 1 for the manufacture of solder resists or primary resists.

## Revendications

1. Utilisation d'époxyacrylates de formule III 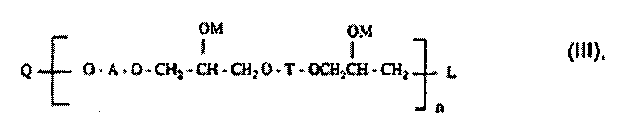 dans laquelle
Q représente l'hydrogène ou un groupe de formules 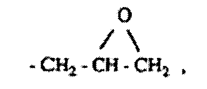 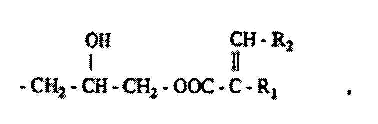 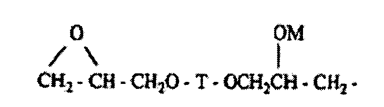 ou 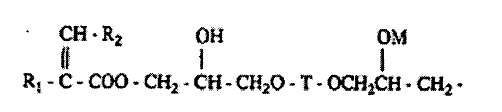 R₁ représente -H ou -CH₃, R₂ représente -H, -CH₃ ou un reste phényle
T est le reste d'un composé aromatique bifonctionnel et
les restes M représentent, indépendamment l'un de l'autre, l'hydrogène ou un groupe de formules 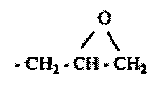 ou 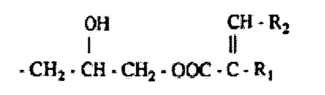
où R₁ et R₂ ont la définition indiquée ci-dessus,
A est le reste d'un composé aromatique bifonctionnel,
n représente un nombre entier de 0 à 300, et
L représente un groupe de formules 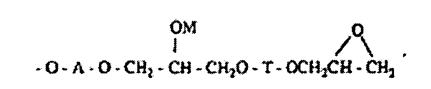 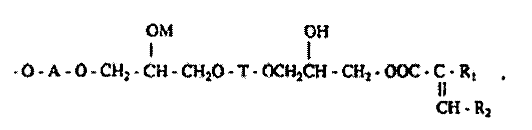
ou -O-A-OM,
les restes M dans la formule III ne pouvant pas tous représenter en même temps de l'hydrogène ou un groupe de formule 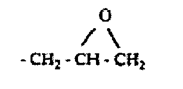 mais au moins 10 moles %, avantageusement 20 à 100 moles % des restes M qui ne se trouvent pas dans les groupes terminaux Q et L représentent un groupe de formule 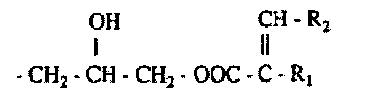 comme composants acrylate dans des formulations de résist.

2. Utilisation suivant la revendication 1, dans laquelle, dans la formule III, R₁ représente l'hydrogène ou un reste méthyle et R₂ représente l'hydrogène, un reste méthyle ou phényle.

3. Utilisation suivant la revendication 1, dans laquelle n représente un nombre entier de 0 à 50 dans la formule III.

4. Utilisation suivant la revendication 1, dans laquelle, dans la formule III, n est égal à 0, Q représente -H et L représente un groupe de formule 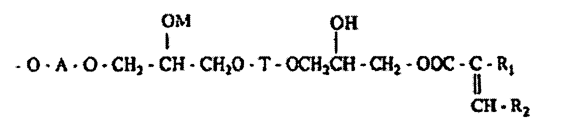 où A, T, R₁ et R₂ ont les définitions indiquées dans la revendication 1.

5. Utilisation suivant la revendication 1, dans laquelle, dans la formule III, A et T répondent aux formules 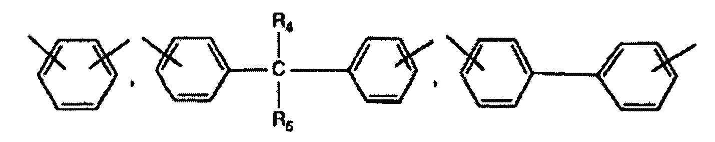 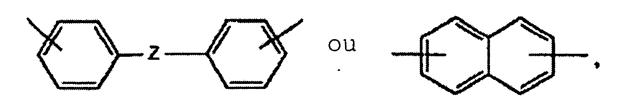 R₄ et R₅ représentent, indépendamment l'un de l'autre, -H ou un reste alkyle en C₁ à C₄, Z représente -S-, -O- ou -SO₂, et les restes aromatiques du chaînon pontal A ou T ne sont pas substitués ou sont substitués avec un halogène ou un reste alkyle en C₁ à C₄.

6. Utilisation suivant la revendication 5, dans laquelle R₄ et R₅ représentent -CH₃ et l'halogène est le brome.

7. Utilisation suivant la revendication 5, dans laquelle A et T dans la formule III répondent à la formule 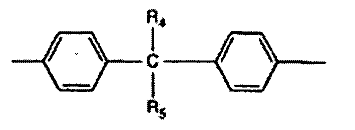 dans laquelle R₄ et R₅ ont les définitions indiquées dans la revendication 6 et les restes phényle du chaînon pontal ne sont pas substitués ou sont substitués avec du brome.

8. Utilisation suivant la revendication 5, dans laquelle A et T correspondent aux formules

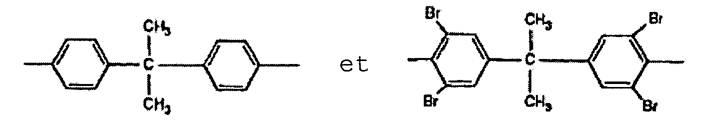

9. Utilisation d'époxyacrylates suivant la revendication 1 pour la fabrication de résists de soudure ou de résists primaires.
